# EUROPÄISCHE PATENTANMELDUNG

(11) **EP 2 826 483 A2**
(43) Veröffentlichungstag der Anmeldung: **21.01.2015**
(21) Anmeldenummer: 14177123.8
(22) Anmeldetag: 15.07.2014
(51) Int. Cl.: A61K 31/7004, A61P 7/02, A61K 31/198, A61K 31/401, A61K 31/4045, A23L 1/305, A23L 1/29

(54) **Stoffgemisch enthaltend Ribose und Aminosäuren zur Behandlung von Sepsis, Koagulopathien, Knochenheilungsstörungen und/oder Knochenblutungen**

(30) Priorität: 15.07.2013 DE 202013006354 U
(71) Anmelder: Fond Livsdraben, 8462 Harlev (DK)
(72) Erfinder: Brøndlund, Marianne, 8462 Harlev (DK)
(74) Vertreter: Laufhütte, Dieter

(57) **Zusammenfassung**

Die Erfindung betrifft ein Stoffgemisch enthaltend Ribose und die Aminosäuren Alanin und Glycin zur Verwendung als Arzneimittel oder Nahrungsergänzungsmittel zur Behandlung von Sepsis, Koagulopathien, Knochenheilungsstörungen und/oder Knochenblutungen. Die Erfindung betrifft ferner ein Stoffgemisch enthaltend Ribose und die Aminosäuren Glycin, Prolin, Lysin, Arginin und Asparagin zur Verwendung als Arzneimittel oder Nahrungsergänzungsmittel zur Behandlung von Sepsis, Koagulopathien, Knochenheilungsstörungen und/oder Knochenblutungen. Des Weiteren betrifft die Erfindung eine Infusions- oder Trinklösung zur Verwendung als Arzneimittel oder Nahrungsergänzungsmittel zur Behandlung von Sepsis, Koagulopathien, Knochenheilungsstörungen und/oder Knochenblutungen, dadurch gekennzeichnet, dass die Infusions- oder Trinklösung die Inhaltsstoffe eines erfindungsgemäßen flüssigen Stoffgemisches in 10-facher bis 100-facher, vorteilhafterweise in 25-facher bis 50-facher Verdünnung enthält.

## Beschreibung

Die Erfindung betrifft ein Stoffgemisch zur Verwendung als Arzneimittel oder Nahrungsergänzungsmittel zur Behandlung von Sepsis, Koagulopathien, Knochenheilungsstörungen und/oder Knochenblutungen.

Die Behandlung von durch Sepsis induzierten Koagulopathien gestaltet sich mit den im Stand der Technik bekannten Mitteln schwierig. Bei Sepsis treten durch die Überlastung des Blutes mit biologischen Abfallprodukten Koagulopathien auf, wobei das Blut einerseits nicht mehr richtig gerinnt, andererseits aber dennoch Thrombosen bildet (disseminierte intravasale Gerinnung). Daher befindet man sich weitestgehend in einer medikamentösen Zwickmühle.

Auch Knochenheilungsstörungen sind ein in der medizinischen Praxis auftretendes Problem, für das Abhilfe geschaffen werden soll.

In der DE 10 2011 105 105 594 A1 wird ein Stoffgemisch zur Verwendung als Arzneimittel oder Nahrungsergänzungsmittel offenbart, das zur Behandlung für eine Reihe medizinischer Indikationen angedacht war.

Es hat sich nun auf Grund neuer experimenteller Erkenntnisse herausgestellt, dass das dort offenbarte Stoffgemisch ausgezeichnete Effekte in der Behandlung von Sepsis und/oder Koagulopathien, insbesondere von durch Sepsis induzierten Koagulopathien aufweist.

Ferner hat sich gezeigt, dass das dort offenbarte Gemisch zur Behandlung von Knochenheilungsstörungen eingesetzt werden kann.

Im Zusammenhang mit allen im Folgenden diskutierten Ausführungsbeispielen gilt, dass das Stoffgemisch die genannten Bestandteile enthalten kann oder daraus bestehen kann. Zweiteres ist bevorzugt, denn die Wirkung kann ausschließlich auf dieser Grundlage erzielt werden und zusätzliche natürliche oder synthetische Wirkstoffe, die gegebenenfalls den Organismus belasten könnten oder unerwünschte Nebenwirkungen hervorrufen könnten, sind nicht erforderlich. Vorteilhafterweise enthält das Stoffgemisch ausschließlich natürliche Inhaltsstoffe.

Ferner gilt im Zusammenhang mit allen im folgenden diskutierten Ausführungsbeispielen, dass die Bestandteile, insbesondere die Aminosäuren in einer bevorzugen Ausführungsform solche sind, die rekristallisiert sind, d.h. durch ein Rekristallisationsverfahren verändert und/oder gereinigt wurden. Die Rekristallisation kann dabei in Wasser, wässriger Lösung oder einem anderen flüssigen Medium erfolgen, wobei die Rekristallisation in Wasser oder einer wässrigen Lösung besonders bevorzugt ist. Vorzugsweise erfolgt die Rekristallisation dabei mehrere Male, beispielsweise zwischen 2x und 10x, weiter vorzugsweise genau 7x.

Gemäß Anspruch 1 betrifft die Erfindung ein Stoffgemisch enthaltend Ribose und die Aminosäuren Alanin und Glycin zur Verwendung als Arzneimittel oder Nahrungsergänzungsmittel zur Behandlung von Sepsis und/oder Koagulopathien, insbesondere von durch Sepsis induzierten Koagulopathien. Eine spezifische Indikation, die behandelt werden kann, ist beispielsweise die disseminierte intravasale Gerinnung. Denkbar ist dabei der Einsatz zur Behandlung bzw. unterstützenden Behandlung der genannten Indikationen.

Gemäß Anspruch 2 betrifft die Erfindung ein Stoffgemisch enthaltend Ribose und die Aminosäuren Alanin und Glycin zur Verwendung als Arzneimittel oder Nahrungsergänzungsmittel zur Behandlung von Knochenheilungsstörungen und/oder Knochenblutungen.

In einer Ausführungsform ist die verwendete Ribose D-Ribose. Diese stellt einen wichtigen Baustein im menschlichen Körper, beispielsweise in der RNA-Backbone und als Bestandteil des Adenosins, und damit des ATP, ADP, AMP oder cAMP.

In einer Ausführungsform ist das verwendete Alanin razemisches (DL)-α-Alanin. Alanin kann als Bestandteil von Infusions- oder Trinklösungen zur parenteralen Ernährung dienen. Glycin ist als Aminosäure wichtiger Bestandteil nahezu aller Proteine und ein wichtiger Knotenpunkt im Stoffwechsel.

Das relative Massenverhältnis von Ribose, Alanin und Glycin im erfindungsgemäßen Stoffgemisch beträgt in einer Ausführungsform A:B:C mit 35<A<200, 20<B<80 und 1<C<40. In einer bevorzugten Ausführungsform beträgt das relative Massenverhältnis A:B:C mit 60<A<120, 30<B<70 und 2<C<20, weiterhin vorteilhafterweise 50<A<90, 30<B<50 und 3<C<5. Besonders bevorzugt ist ein Massenverhältnis von etwa 70:40:4.

In einer ersten Ausführungsform kann das Stoffgemisch weiterhin Glutamin enthalten, insbesondere L-Glutamin. Vorteilhafterweise beträgt das relative Massenverhältnis von Ribose, Alanin, Glycin und Glutamin dabei A:B:C:D mit 35 < A < 200, 20 < B < 80, 1 < C < 40 und 20 < D < 80 beträgt, vorteilhafterweise mit 60<A<120, 30< B <70, 2 < C < 20 und 30 < D < 60, weiterhin vorteilhafterweise mit A = 70, B = 40, C = 4 und D = 50.

Das Stoffgemisch kann insbesondere in der ersten Ausführungsform weiterhin einen oder mehrere der folgenden Stoffe enthalten: Serin, Valin, Leucin, Isoleucin, Cystein, 5-Hydroxy-Tryptophan, Tryptophan. Vorteilhafterweise handelt es sich dabei jeweils um L-Serin, L-Valin, L-Leucin, L-Isoleucin, L-Cystein, L-5-Hydroxy-Tryptophan, L-Tryptophan.

Enthält das Stoffgemisch einen oder mehrere dieser Stoffe, so beträgt vorteilhafterweise das relative Massenverhältnis von Ribose, Alanin, Glycin und dem jeweiligen Stoff A:B:C:D mit 35 < A < 200, 20 < B < 80 und 1 < C < 40, vorteilhafterweise mit 60<A<120, 30<B<70 und 2<C<20, weiterhin vorteilhafterweise mit A = 70, B = 40 und C = 4, wobei D für den jeweiligen Stoff, wenn er in der Stoffmischung enthalten ist, vorteilhafterweise folgenden Wert hat:
- Serin 10 < D < 150, vorteilhafterweise 40 < D < 120, weiterhin vorteilhafterweise D = 100;
- Valin 40 < D < 150, vorteilhafterweise 60 < D < 120, weiterhin vorteilhafterweise D = 70;
- Leucin 5 < D < 80, vorteilhafterweise 10 < D < 40, weiterhin vorteilhafterweise D = 30;
- Isoleucin 1 < D < 80, vorteilhafterweise 1 < D < 60, weiterhin vorteilhafterweise D = 40;
- Cystein 1 < D < 150, vorteilhafterweise 5 < D < 120, weiterhin vorteilhafterweise D = 10;
- 5-Hydroxy-Tryptophan 1 < D < 100, vorteilhafterweise 10 < D < 60, weiterhin vorteilhafterweise D = 50;
- Tryptophan 1 < D < 100, vorteilhafterweise 10 < D < 60, weiterhin vorteilhafterweise D = 50.

Das Stoffgemisch kann dabei 5-Hydroxy-Tryptophan, Tryptophan oder Mischungen aus 5-Hydroxy-Tryptophan und Tryptophan enthalten, wobei vorteilhafterweise der Gesamtanteil an 5-Hydroxy-Tryptophan und Tryptophan 1 < D < 100, vorteilhafterweise 10 < D < 60, weiterhin vorteilhafterweise D = 50 beträgt.

Das Stoffgemisch kann in einer zweiten Ausführungsform weiterhin einen oder mehrere der folgenden Stoffe enthalten: Asparagin, Arginin, Prolin, Lysin. Insbesondere kann das Stoffgemisch alle diese Stoffe enthalten. Vorteilhafterweise handelt es sich bei den Stoffen um L-Asparagin, L-Arginin, L-Prolin und L-Lysin.

Enthält das Stoffgemisch einen oder mehrere dieser Stoffe, so beträgt vorteilhafterweise das relative Massenverhältnis von Ribose, Alanin, Glycin und dem jeweiligen Stoff A:B:C:D mit 35 < A < 200, 20 < B < 80 und 1 < C < 40 beträgt, vorteilhafterweise mit 60<A<120, 30<B<70 und 2<C<20, weiterhin vorteilhafterweise mit A = 70, B = 40 und C = 4, und für:
- Asparagin 1 < D < 100, vorteilhafterweise 10 < D < 80, weiterhin vorteilhafterweise 20 <= D <= 70;
- Arginin: 1 < D < 100, vorteilhafterweise 2 < D < 60, weiterhin vorteilhafterweise 4 <= D <= 40;
- Prolin 1 < D < 100, vorteilhafterweise 5 < D < 80, weiterhin vorteilhafterweise 10 <= D <= 70;
- Lysin 10 < D < 80, vorteilhafterweise 30 < D < 50, weiterhin vorteilhafterweise D = 40.

Vorteilhafterweise enthält das Stoffgemisch gemäß der zweiten Ausführungsform dabei auch die zusätzlichen Stoffe, welche das Stoffgemisch gemäß der ersten Ausführungsform enthält, vorteilhafterweise in den zur ersten Ausführungsform angegebenen Konzentrationen.

Vorteilhafterweise enthält das erfindungsgemäße Stoffgemisch andere Aminosäuren und/oder Zucker nur in einem Massenverhältnis von weniger als 1:10 zu dem Gesamtanteil von Ribose, Alanin und Glycin, weiterhin vorteilhafterweise von weniger als 1:100, weiterhin vorteilhafterweise von weniger als 1:1000. Bevorzugt enthält das erfindungsgemäße Stoffgemisch keine anderen Aminosäuren und/oder Zucker.

In einer Ausführungsform handelt es sich bei dem erfindungsgemäßen Stoffgemisch um ein festes Stoffgemisch, das die Bestandteile als Feststoffe enthält. Ribose, Alanin und Glycin liegen bei Raumtemperatur als Feststoff, typischerweise als weißes Pulver vor.

In einer Ausführungsform ist das erfindungsgemäße, feste Stoffgemisch ein pulverförmiges Gemisch der pulverförmigen Bestandteile Ribose, Alanin sowie Glycin sowie ggf. weiterer Stoffe. In einer Ausführungsform besteht das erfindungsgemäße, feste Stoffgemisch im Wesentlichen oder ausschließlich aus diesen Bestandteilen.

In einer Ausführungsform enthält ein Behältnis genau eine Dosierungseinheit, die zwischen etwa 35 g und 200 g Ribose, zwischen etwa 20 g und 80 g Alanin und zwischen etwa 1 g und 40 g Glycin enthält. Dies stellt eine geeignete Dosierung hinsichtlich der Stoffmengen und insbesondere der Stoffmengenverhältnisse bei einer weiteren Verwendung zur Herstellung eines Nahrungsergänzungsmittels, einer ergänzenden bilanzierten Diät oder eines Medikaments, beispielsweise durch Auflösen und gegebenenfalls Verdünnen, dar.

Eine bevorzugte Menge an Ribose innerhalb einer Dosierungseinheit liegt zwischen etwa 60 g und 120 g, vorteilhafterweise zwischen 50 g und 90 g. Eine bevorzugte Menge an Alanin innerhalb einer Dosierungseinheit liegt zwischen etwa 30 g bis 70 g, vorteilhafterweise zwischen 30 g und 50 g. Eine bevorzugte Menge an Glycin innerhalb einer Dosierungseinheit liegt zwischen 2 g und 20 g, vorteilhafterweise zwischen 3 g und 5 g Glycin.

Die Dosierungseinheiten können gemäß der ersten Ausführungsform weiterhin einen oder mehrere der Stoffe Glutamin, Serin, Valin, Leucin, Isoleucin, Cystein, 5-Hydroxy-Tryptophan, Tryptophan enthalten, insbesondere in einer Menge, dass das Stoffgemisch in der Dosierungseinheit eine der oben näher beschriebenen Gesamtzusammensetzungen aufweist.

Die Dosierungseinheiten können gemäß der zweiten Ausführungsform weiterhin einen oder mehrere der Stoffe Asparagin, Arginin, Prolin, Lysin enthalten, insbesondere in einer Menge, dass das Stoffgemisch in der Dosierungseinheit eine der oben näher beschriebenen Gesamtzusammensetzungen aufweist.

Die Stoffe könnend dabei auch in mehreren Dosierungseinheiten zur Verfügung gestellt werden.

In einer Ausführungsform handelt es sich bei dem erfindungsgemäßen Stoffgemisch um ein flüssiges Stoffgemisch, das die Bestandteile in wässriger Lösung enthält. Ribose, Alanin und Glycin sind gut wasserlöslich, wodurch diese problemlos bei Raumtemperatur in Wasser oder wässrigen Lösungen aufgelöst werden können.

In einer bevorzugten Ausführungsform enthält ein erfindungsgemäßes, flüssiges Stoffgemisch die Bestandteile in Lösung in Wasser oder einer isotonischen Lösung. Insbesondere kann energetisiertes Wasser eingesetzt werden, bevorzugt energetisiertes Wasser nach Mee-Hu. Auch der Einsatz anderer physiologisch verträglicher Lösungen ist denkbar.

In einer bevorzugten Ausführungsform enthält ein erfindungsgemäßes, flüssiges Stoffgemisch zwischen etwa 35 g/l und etwa 200 g/l Ribose, zwischen etwa 20 g/l und etwa 80 g/l Alanin, zwischen etwa 1 g/l und etwa 40 g/l Glycin.

Ein bevorzugter Konzentrationsbereich für Ribose liegt zwischen etwa 60 g/l und 120 g/l, insbesondere zwischen 50 g/l und etwa 90 g/l. Ein bevorzugter Konzentrationsbereich für Alanin liegt zwischen etwa 30 g/l und 70 g/l, insbesondere zwischen 30 g/l und etwa 50 g/l. Ein bevorzugter Konzentrationsbereich für Glycin liegt zwischen etwa 2 g/l und 20 g/l, insbesondere zwischen 3 g/l und etwa 5 g/l.

Weiterhin kann die wässrige Lösung gemäß der ersten Ausführungsform vorteilhafterweise einen oder mehrere der folgenden Stoffe enthalten: Glutamin, Serin, Valin, Leucin, Isoleucin, Cystein, 5-Hydroxy-Tryptophan, Tryptophan.

Bevorzugte Konzentrationsbereiche für die jeweiligen Stoffe, soweit sie in der wässrigen Lösung vorhanden sind, betragen dabei wie folgt:
- Glutamin zwischen 20 g/l und 80 g/l, vorteilhafterweise zwischen 30 g/l und 60 g/l, weiterhin vorteilhafterweise 50 g/l;
- Serin: zwischen 10 g/l und 150 g/l, vorteilhafterweise zwischen 40 g/l und 120 g/l, weiterhin vorteilhafterweise 100 g/l;
- Valin zwischen 40 g/l und 150 g/l, vorteilhafterweise zwischen 60 g/l und 120 g/l, weiterhin vorteilhafterweise 70 g/l;
- Leucin zwischen 5 g/l und 80 g/l, vorteilhafterweise zwischen 10 g/l und 40 g/l, weiterhin vorteilhafterweise 30 g/l;
- Isoleucin zwischen 1 g/l und 80 g/l, vorteilhafterweise zwischen 1 g/l und 60 g/l, weiterhin vorteilhafterweise 40 g/l;
- Cystein zwischen 1 g/l und 150 g/l, vorteilhafterweise zwischen 5 g/l und 120 g/l, weiterhin vorteilhafterweise 10 g/l;
- 5-Hydroxy-Tryptophan zwischen 1 g/l und 100 g/l, vorteilhafterweise zwischen 10 g/l und 60 g/l, weiterhin vorteilhafterweise 50 g/l;
- Tryptophan zwischen 1 g/l und 100 g/l, vorteilhafterweise zwischen 10 g/l und 60 g/l, weiterhin vorteilhafterweise 50 g/l.

Das Stoffgemisch kann dabei 5-Hydroxy-Tryptophan, Tryptophan oder Mischungen aus 5-Hydroxy-Tryptophan und Tryptophan enthalten, wobei vorteilhafterweise die Gesamtkonzentration an 5-Hydroxy-Tryptophan und Tryptophan zwischen 1 g/l und 100 g/l, vorteilhafterweise zwischen 10 g/l und 60 g/l, weiterhin vorteilhafterweise 50 g/l beträgt.

Weiterhin kann die wässrige Lösung gemäß der zweiten Ausführungsform vorteilhafterweise einen oder mehrere der folgenden Stoffe enthalten: Asparagin, Arginin, Prolin, Lysin.

Bevorzugte Konzentrationsbereiche für die jeweiligen Stoffe, soweit sie in der wässrigen Lösung vorhanden sind, betragen dabei wie folgt:
- Asparagin zwischen 1 g/l und 100 g/l, vorteilhafterweise zwischen 10 g/l und 80 g/l, weiterhin vorteilhafterweise zwischen 20 g/l und 70 g/l;
- Arginin zwischen 1 g/l und 100 g/l, vorteilhafterweise zwischen 2 g/l und 60 g/l, weiterhin vorteilhafterweise zwischen 4 g/l und 40 g/l;
- Prolin zwischen 1 g/l und 100 g/l, vorteilhafterweise zwischen 5 g/l und 80 g/l, weiterhin vorteilhafterweise zwischen 10 g/l und 70 g/l;
- Lysin zwischen 10 g/l und 80 g/l, vorteilhafterweise zwischen 30 g/l und 50 g/l, weiterhin vorteilhafterweise 40 g/l.

Vorteilhafterweise enthält die wässrige Lösung gemäß der zweiten Ausführungsform dabei auch die in der wässrigen Lösung gemäß der ersten Ausführungsform enthaltenen Stoffe in der dort angegebenen Konzentration.

In einer weiteren alternativen Ausführungsform kann vorgesehen sein, dass ein erfindungsgemäßes flüssiges Stoffgemisch im Wesentlichen oder ausschließlich aus den Bestandteilen Ribose, Alanin und Glycin und Wasser bzw. einer physiologisch verträglichen Lösung, insbesondere einer isotonischen Lösung besteht. Insbesondere kann energetisiertes Wasser eingesetzt werden, bevorzugt energetisiertes Wasser nach Mee-Hu.

Ferner betrifft die Erfindung ein Stoffgemisch enthaltend Ribose und die Aminosäuren Glycin, Prolin, Lysin, Arginin und Asparagin zur Verwendung als Arzneimittel oder Nahrungsergänzungsmittel zur Behandlung von Sepsis und/oder Koagulopathien, insbesondere von durch Sepsis induzierten Koagulopathien. Eine spezifische Indikation, die behandelt werden kann, ist beispielsweise die disseminierte intravasale Gerinnung. Denkbar ist dabei der Einsatz zur Behandlung bzw. unterstützenden Behandlung der genannten Indikationen.

Weiterhin betrifft die Erfindung ein Stoffgemisch enthaltend Ribose und die Aminosäuren Glycin, Prolin, Lysin, Arginin und Asparagin zur Verwendung als Arzneimittel oder Nahrungsergänzungsmittel zur Behandlung von Knochenheilungsstörungen und/oder Knochenblutungen.

In einer Ausführungsform ist die verwendete Ribose D-Ribose. Diese stellt einen wichtigen Baustein im menschlichen Körper, beispielsweise in der RNA-Backbone und als Bestandteil des Adenosins, und damit des ATP, ADP, AMP oder cAMP. Glycin ist als Aminosäure wichtiger Bestandteil nahezu aller Proteine und ein wichtiger Knotenpunkt im Stoffwechsel. Bei Prolin, Lysin, Arginin und Asparagin handelt es sich ebenfalls um wichtige Aminosäuren. Vorteilhafterweise wird dabei L-Glycin, L-Prolin, L-Lysin, L-Arginin und L-Asparagin eingesetzt.

In einer bevorzugten Aufführungsform beträgt das relative Massenverhältnis von Ribose, Glycin, Prolin, Lysin, Arginin und Asparagin A:B:C:D:E:F, mit den folgenden Werten:
- Ribose: 35 < A < 200, vorteilhafterweise 60<A<120, weiterhin vorteilhafterweise A = 70;
- Glycin: 1 < B < 40, vorteilhafterweise 5 < B < 20, weiterhin vorteilhafterweise B = 10;
- Prolin: 10 < C < 100, vorteilhafterweise 20 < C < 80, weiterhin vorteilhafterweise 40 <= C <= 60;
- Lysin: 20 < D < 80, vorteilhafterweise 30 < D < 60, weiterhin vorteilhafterweise D = 40;
- Arginin: 1 < E < 100, vorteilhafterweise 2 < E < 60, weiterhin vorteilhafterweise 4 <= E <= 40;
- Asparagin: 10 < F < 80, vorteilhafterweise 20 < F < 50, weiterhin vorteilhafterweise F = 30.

In einer Ausführung enthält das erfindungsgemäße Stoffgemisch andere Aminosäuren und/oder Zucker nur in einem Massenverhältnis von weniger als 1:10 zu dem Gesamtanteil von Ribose, Glycin, Prolin, Lysin, Arginin und Asparagin, weiterhin vorteilhafterweise von weniger als 1:100, weiterhin vorteilhafterweise von weniger als 1:1000. Insbesondere kann das erfindungsgemäße Stoffgemisch keine anderen Aminosäuren und/oder Zucker enthalten.

In einer Ausführungsform handelt es sich bei dem erfindungsgemäßen Stoffgemisch um ein festes Stoffgemisch, das die Bestandteile als Feststoffe enthält.

In einer Ausführungsform ist das erfindungsgemäße, feste Stoffgemisch ein pulverförmiges Gemisch der pulverförmigen Bestandteile Ribose, Glycin, Prolin, Lysin, Arginin und Asparagin. In einer Ausführungsform besteht das erfindungsgemäße, feste Stoffgemisch im Wesentlichen oder ausschließlich aus diesen Bestandteilen.

In einer Ausführungsform enthält ein Behältnis genau eine Dosierungseinheit, welche in einem Liter Flüssigkeit aufgelöst die unten im Hinblick auf ein flüssiges Stoffgemisch angegebenen bevorzugten Konzentrationsbereiche ergibt.

In einer Ausführungsform handelt es sich bei dem erfindungsgemäßen Stoffgemisch um ein flüssiges Stoffgemisch, das Ribose, Glycin, Prolin, Lysin, Arginin und Asparagin in wässriger Lösung enthält.

In einer bevorzugten Ausführungsform enthält ein erfindungsgemäßes, flüssiges Stoffgemisch die Bestandteile in Lösung in Wasser oder einer isotonischen Lösung. Insbesondere kann energetisiertes Wasser eingesetzt werden, bevorzugt energetisiertes Wasser nach Mee-Hu. Auch der Einsatz anderer physiologisch verträglicher Lösungen ist denkbar.

In einer bevorzugten Ausführungsform enthält ein erfindungsgemäßes, flüssiges Stoffgemisch den jeweiligen Stoff in folgender Konzentration:
- Ribose zwischen 35 g/l und 200 g/l, vorteilhafterweise zwischen 60<A<120, weiterhin vorteilhafterweise A = 70;
- Glycin zwischen 1 g/l und 40 g/l, vorteilhafterweise zwischen 5 g/l und 20 g/l, weiterhin vorteilhafterweise B = 10 g/l;
- Prolin zwischen 10 g/l und 100 g/l, vorteilhafterweise zwischen 20 g/l und 80 g/l, weiterhin vorteilhafterweise zwischen 40 g/l und 60 g/l;
- Lysin zwischen 20 g/l und 80 g/l, vorteilhafterweise zwischen 30 g/l und 60 g/l, weiterhin vorteilhafterweise D = 40 g/l;
- Arginin zwischen 1 g/l und 100 g/l, vorteilhafterweise zwischen 2 g/l und 60 g/l, weiterhin vorteilhafterweise zwischen 4 g/l und 40 g/l;
- Asparagin zwischen 10 g/l und 80 g/l, vorteilhafterweise zwischen 20 g/l und 50 g/l, weiterhin vorteilhafterweise F = 30 g/l.

In einer bevorzugten Ausführungsform enthält ein erfindungsgemäßes, flüssiges Stoffgemisch ferner kolloidales Silber oder Gold oder ein Gemisch daraus. Das kolloidale Silber oder Gold oder Gemisch daraus kann in einer Menge von zwischen 3 und 7 ppm und vorzugsweise von 5 ppm gemessen an der Gesamtmasse des flüssigen Stoffgemischs vorhanden ist.

Bei dem erfindungsgemäßen, flüssigen Stoffgemisch kann es sich bei allen oben beschriebenen Ausführungsformen um ein Konzentrat handeln. Dies kann zum

Erhalt des verabreichungsfertigen Nahrungsergänzungsmittels oder Medikaments beispielsweise weiter mit Wasser oder physiologischer Kochsalzlösung verdünnt werden. Die oben genannten Mischungsverhältnisse im Konzentrat sind dabei bereits so gewählt, dass durch eine bloße Verdünnung die gewünschten Stoffmengenverhältnisse erreicht werden. Weiterhin kann zur Verdünnung energetisiertes Wasser nach Mee-Hu eingesetzt werden.

Die Erfindung betrifft ferner ein Verfahren zur Bereitstellung eines erfindungsgemäßen, flüssigen Stoffgemisches, wobei ein erfindungsgemäßes, festes Stoffgemisch in Wasser oder einer anderen physiologisch verträglichen wässrigen Lösung aufgelöst wird. Vorteilhafterweise wird dabei energetisiertes Wasser nach Mee-Hu eingesetzt.

Der Lösevorgang kann bei Raumtemperatur (ca. 21°C) und/oder unter vorsichtigem Rühren und/oder unter sterilen Bedingungen erfolgen.

Beispielsweise kann eine Dosierungseinheit eines erfindungsgemäßen festen Stoffgemisches in zwischen etwa 500 ml und etwa 1500 ml, vorzugsweise in zwischen etwa 800 ml und etwa 1200 ml und weiter vorzugsweise in ungefähr oder genau 1000 ml Wasser, physiologischer Kochsalzlösung oder einer anderen physiologisch verträglichen wässrigen Lösung aufgelöst werden. Vorteilhafterweise wird dabei energetisiertes Wasser nach Mee-Hu eingesetzt.

Da nicht alle der ggf. zusätzlich vorgesehenen Stoffe leicht löslich sind und die Gesamtmenge an gelösten Stoffen ggf. groß werden kann, wird weiterhin ein Verfahren zur Herstellung einer wässrigen gemäß der Erfindung Lösung überlegt, bei welchem die Lösung durch Mischung zweier Teillösungen hergestellt wird. Dabei wird eine erste Teillösung hergestellt, indem einer wässrigen Lösung einer oder mehrere der Stoffe Valin, Leucin, Isoleucin, 5-Hydroxy-Tryptophan, Tryptophan unter Rühren zugegeben werden. Weiterhin wird eine zweite Teillösung hergestellt wird, indem einer wässrigen Lösung ggf. Glutamin und einer oder mehrere der Stoffe Cystein, Serin, Alanin, Glycin unter Rühren zugegeben werden. Dann werden die erste und die zweite Teillösung gemischt. Vorteilhafterweise erfolgt das Mischen, indem die erste Teillösung der zweiten Teillösung beigemischt wird, vorteilhafterweise durch ein langsames Beifügen der ersten Teillösung unter ständigem Rühren. Weiterhin kann der hierdurch entstandenen Lösung Ribose beigefügt werden. Insbesondere kann das Verfahren zur Herstellung einer wässrigen Lösung gemäß der ersten Ausführungsform eingesetzt werden.

Hierbei werden zunächst die schwer lösbaren Stoffe in der ersten Teillösung gelöst, dann ggf. Glutamin und die leicht lösbaren Stoffe in einer zweiten Teillösung gelöst, welche dann gemischt werden, wobei zuletzt ggf. die Ribose zugegeben wird. Die Lösung erfolgt dabei vorteilhafterweise unter ständigem Rühren, und insbesondere Stoff für Stoff aufeinanderfolgend.

Vorteilhafterweise werden dabei die oben genannten Stoffe in einer Menge zugegeben, dass sich ein erfindungsgemäßes Stoffgemisch gemäß der ersten Ausführungsform ergibt, wie sie oben beschrieben wurde, insbesondere eine wässrige Lösung gemäß der ersten Ausführungsform, wie sie oben beschrieben wurde.

In einer weiteren Ausführungsform werden der fertigen Lösung einer oder mehrere der Stoffe Asparagin, Arginin, Prolin, Lysin beigefügt. Insbesondere kann das Verfahren dabei zur Herstellung einer wässrigen Lösung gemäß der zweiten Ausführungsform eingesetzt werden.

Die vorliegende Erfindung betrifft weiterhin ein Verfahren zur Herstellung einer wässrigen Lösung, bei welchem zunächst einer oder mehrere der Stoffe Valin, Leucin, Isoleucin, 5-Hydroxy-Tryptophan, Tryptophan, Glutamin, Cystein, Serin, Alanin, Glycin bei einer höheren Temperatur der wässrigen Lösung gelöst werden, woraufhin die Temperatur der wässrigen Lösung gesenkt und Ribose und/oder einer oder mehrere der Stoffe Asparagin, Arginin, Prolin, Lysin bei niedrigerer Temperatur gelöst wird. Zunächst wird daher erfindungsgemäß eine höhere Temperatur genutzt, um eine möglichst gute Lösbarkeit zu erreichen, während Ribose bei niedrigerer Temperatur zugegeben wird, um diese nicht zu beschädigen.

Vorteilhafterweise werden dabei einer oder mehrere der Stoffe Valin, Leucin, Isoleucin, 5-Hydroxy-Tryptophan, Tryptophan, Glutamin, Cystein, Serin, Alanin, Glycin bei einer Temperatur der wässrigen Lösung zwischen 50°C und 80°C, vorteilhafterweise zwischen 60°C und 75°C, weiterhin vorteilhafterweise bei 70°C gelöst, und/oder die Ribose bei einer Temperatur zwischen 20°C und 50°C, vorteilhafterweise zwischen 30°C und 45°C, weiterhin vorteilhafterweise bei 40°C.

Ggf. kann dann zur Herstellung einer Lösung gemäß der zweiten Ausführungsform einer oder mehrere der Stoffe Asparagin, Arginin, Prolin, Lysin zugegeben werden.

Vorteilhafterweise wird dabei das soeben beschriebene Verfahren der Lösung bei unterschiedlichen Temperaturen mit der weiter oben beschriebenen Herstellung aus Teillösungen kombiniert.

Insbesondere werden dabei die erste und die zweite Teillösung unter höherer Temperatur hergestellt und gemischt. Die gemischte Lösung wird dann abgekühlt, und die Ribose beigefügt. Ggf. kann dann zur Herstellung einer Lösung gemäß der zweiten Ausführungsform einer oder mehrere der Stoffe Asparagin, Arginin, Prolin, Lysin zugegeben werden.

Die Erfindung betrifft des Weiteren eine Trinklösung, die die Inhaltsstoffe eines erfindungsgemäßen, flüssigen Stoffgemisches in etwa 10-facher bis 100-facher, vorteilhafterweise 20-facher bis etwa 60-facher Verdünnung enthält. Vorzugsweise enthält eine erfindungsgemäße Trinklösung diese Inhaltsstoffe in 25-facher oder etwa 50-facher Verdünnung. Als Basis für die Trinklösung dient vorzugsweise energetisiertes Wasser nach Mee-Hu. Auch der Einsatz anderer physiologisch verträglicher Lösungen ist denkbar.

Die Erfindung betrifft des Weiteren eine Infusionslösung, die die Inhaltsstoffe eines erfindungsgemäßen, flüssigen Stoffgemisches in etwa 10-facher bis 100-facher, vorteilhafterweise 20-facher bis etwa 60-facher Verdünnung enthält. Vorzugsweise enthält eine erfindungsgemäße Infusionslösung diese Inhaltsstoffe in 25-facher oder etwa 50-facher Verdünnung. Als Basis für die Infusionslösung dient vorzugsweise energetisiertes Wasser nach Mee-Hu. Auch der Einsatz anderer physiologisch verträglicher Lösungen ist denkbar. Die Infusionen eigenen sich zur parenteralen oder oralen Verabreichung an einen Patienten.

Je nach der gewünschten Behandlungsintensität sowie dem Zustand des Patienten kann ein behandelnder Arzt den Verdünnungsgrad der als ergänzende bilanzierte Diät dienenden Trinklösung oder Infusion innerhalb der erfinderischen Schranken anpassen.

Die Erfindung betrifft des Weiteren ein Verfahren zur Bereitstellung einer erfindungsgemäßen Trinklösung oder Infusionslösung durch Verdünnung eines erfindungsgemäßen, flüssigen Stoffgemisches in einer Basislösung, vorzugsweise auf Basis von energetisiertem Wasser nach Mee-Hu.

Beispielsweise werden etwa 10 ml (zwischen etwa 8 ml und etwa 12 ml) eines erfindungsgemäßen flüssigen Stoffgemisches in entweder etwa 250 ml oder etwa 500 ml einer Basislösung verdünnt. Als Basislösung dient vorzugsweise steriles Wasser oder eine isotonische Kochsalzlösung (insbesondere wird dabei für die Infusions- oder Trinkbasislösung energetisiertes Wasser nach Mee-Hu eingesetzt). Auch der Einsatz anderer physiologisch verträglicher Lösungen ist denkbar.

Ferner gilt im Zusammenhang mit allen vorstehend beschriebenen Herstellungsverfahren, dass die Bestandteile, insbesondere die Aminosäuren vorgelagert zum Verfahren durch Rekristallisation verändert und/oder gereinigt werden können. Die Rekristallisation kann dabei in Wasser, wässriger Lösung oder einem anderen flüssigen Medium erfolgen, wobei die Rekristallisation in Wasser oder einer wässrigen Lösung besonders bevorzugt ist. Vorzugsweise erfolgt die Rekristallisation dabei mehrere Male, beispielsweise zwischen 2x und 10x, weiter vorzugsweise genau 7x.

Weitere Einzelheiten und Vorteile der Erfindung ergeben sich aus den im folgenden beschriebenen Figuren und Ausführungsbeispielen. In den Figuren zeigen:
- Figur 1:: Küvetten mit Blut einer Patientin mit durch Sepsis hervorgerufener Koagulopathie vor und nach Behandlung mit einem erfindungsgemäßen Stoffgemisch;
- Figur 2:: Weitere derartige Küvetten;
- Figur 3:: Graphische Darstellung der Abnahme des ACS Wertes nach Behandlung mit einem erfindungsgemäßen Stoffgemisch
- Figur 4:: Eine weitere derartige graphische Darstellung;
- Figur 5:: Röntgenbild einer Tibiakopf-Impressionsfraktur einer Patientin;
- Figur 6:: Röntgenbild einer Kalkaneus-Fraktur derselben Patientin;
- Figur 7:: MRT der Tibiakopf-Impressionsfraktur gemäß Figur 5;
- Figur 8:: MRT der Kalkaneus-Fraktur gemäß Figur 6; und
- Figur 9:: MRT der in Figuren 5 und 7 gezeigten Tibiakopf-Impressionsfraktur nach Behandlung mit einem erfindungsgemäßen Stoffgemisch.
- Figur 10:: Graphische Darstellung der Abnahme des ACS Wertes nach Behandlung mit einem weiteren erfindungsgemäßen Stoffgemisch; und
- Figur 11:: Eine weitere derartige graphische Darstellung.

### Herstellungsbeispiel 1:

In einer Verpackungseinheit werden 70 g D-Ribose, 40 g DL-α-Alanin, 4 g Glycin in Pulverform bereitgestellt.

Bei den Aminosäuren DL-α-Alanin und Glycin handelt es sich um durch sechsmalige Rekristallisation veränderte bzw. gereinigte Aminosäuren.

Diese werden in 1 I isotonischer Kochsalzlösung, insbesondere auf Basis von energetisiertem Wasser nach Mee-Hu, bei 21°C unter vorsichtigem Rühren und unter sterilen Bedingungen aufgelöst. Dabei entsteht ein Konzentrat mit 70 g/l D-Ribose, 40 g/l DL-α-Alanin, 4 g/l Glycin und 9 g/l NaCl, welches geeignet und bestimmt ist, in entsprechender Verdünnung zu einer Trinklösung zugesetzt zu werden.

10 ml dieses Konzentrats werden 250 ml oder 500 ml Wasser oder physiologisch verträglicher Lösung, vorzugsweise 0,9%-iger Kochsalzlösung zugesetzt, wobei auch hier vorteilhafterweise energetisiertes Wasser nach Mee-Hu eingesetzt wird.

### Herstellungsbeispiel 2:

In einem zweiten Ausführungsbeispiel können folgende Mengen an Stoffen in 1 I isotonischer Kochsalzlösung, insbesondere auf Basis von energetisiertem Wasser nach Mee-Hu, wie folgt gelöst werden:

Die wässrige Lösung wird auf zwei Gefäße aufgeteilt, um zunächst zwei Teillösungen herzustellen:

### 1. Teillösung ( z.B. mit 50% der Flüssigkeit):

In der ersten Teillösung werden bei einer Temperatur der Kochsalzlösung von 70°C werden unter ständigem Rühren die schwer löslichen Stoffe gelöst:

30g L-Leucin, 40 g L-Isoleucin, 50 g L-5-Hydroxy-Tryptophan oder L-Tryptophan oder eine Mischung der beiden;

### 2. Teillösung ( z.B. mit 50% der Flüssigkeit):

In der zweiten Teillösung werden bei einer Temperatur der Kochsalzlösung von 70°C werden unter ständigem Rühren zunächst
50 g L-Glutamin
und dann die leicht löslichen Stoffe:
40 g DL-α-Alanin, 4 g Glycin, 100 g L-Serin, 100 g L-Cystein
gelöst.

Die erste Teillösung wird dann in die zweite Teillösung eingerührt, vorteilhafterweise durch langsames Zugeben der ersten Teillösung unter Rühren. Das Mischen kann dabei bei einer Temperatur der Lösungen von 70° C erfolgen.

Nun wird die Temperatur der Lösung auf 40°C reduziert und
70 g D-Ribose zugegeben.
10 ml dieses Konzentrats werden wiederum 250 ml oder 500 ml Wasser oder physiologisch verträglicher Lösung, vorzugsweise 0,9%-iger Kochsalzlösung zugesetzt, wobei auch hier vorteilhafterweise energetisiertes Wasser nach Mee-Hu eingesetzt wird.

Je nach Patient können dabei auch alternative Zusammensetzungen eingesetzt werden, wobei Ribose, Alanin, Glycin und ggf. Glutamin eine Basismischung bilden, welcher einer oder mehrerer der Stoffe Serin, Valin, Leucin, Isoleucin, Cystein, 5-Hydroxy-Tryptophan und Tryptophan zugegeben werden können.

Bei allen verwendeten Aminosäuren handelt es sich um durch sechsmalige Rekristallisation veränderte bzw. gereinigte Aminosäuren.

### Herstellungsbeispiel 3:

Das dritte Ausführungsbeispiel basiert auf einem Konzentrat gemäß dem zweiten Ausführungsbeispiel.

Dabei wird 1 I Konzentrat, welches wie oben zum zweiten Ausführungsbeispiel dargestellt hergestellt wird, weiter 20 g, 40 g oder 70 g L-Asparagin, 4 g, 7 g, 10 g, 30 g oder 40 g L-Arginin, 10 g, 30 g oder 70 g L-Prolin und 40 g L-Lysin unter vorsichtigem Rühren und unter sterilen Bedingungen beigemischt.

Dabei entsteht ein Konzentrat, welches geeignet und bestimmt ist, in entsprechender Verdünnung zu einer Trinklösung zugesetzt zu werden.

10 ml dieses Konzentrats werden wiederum 250 ml oder 500 ml Wasser oder physiologisch verträglicher Lösung, vorzugsweise 0,9%-iger Kochsalzlösung zugesetzt, wobei auch hier vorteilhafterweise energetisiertes Wasser nach Mee-Hu eingesetzt wird.

Bei allen verwendeten Aminosäuren handelt es sich um durch sechsmalige Rekristallisation veränderte bzw. gereinigte Aminosäuren.

### Herstellungsbeispiel 4:

In einer oder mehreren Verpackungseinheiten, beispielsweise einem beschichteten Papiersäckchen, werden 70 g D-Ribose, 10 g Glycin, 40 g oder 60 g L-Prolin, 40 g Lysin, 4 g, 7 g, 10 g, 30 g oder 40 g L-Arginin und 30 g L-Asparagin in Pulverform bereitgestellt.

Diese werden in 1 I isotonischer Kochsalzlösung, insbesondere auf Basis von energetisiertem Wasser nach Mee-Hu, bei 21 °C unter vorsichtigem Rühren und unter sterilen Bedingungen aufgelöst. Dabei entsteht ein Konzentrat, welches geeignet und bestimmt ist, in entsprechender Verdünnung zu einer Trinklösung zugesetzt zu werden.

10 ml dieses Konzentrats werden wiederum 250 ml oder 500 ml Wasser oder physiologisch verträglicher Lösung, vorzugsweise 0,9%-iger Kochsalzlösung zugesetzt, wobei auch hier vorteilhafterweise energetisiertes Wasser nach Mee-Hu eingesetzt wird.

Bei allen verwendeten Aminosäuren handelt es sich um durch sechsmalige Rekristallisation veränderte bzw. gereinigte Aminosäuren.

### Anwendungsbeispiel 1

Das erfindungsgemäße Stoffgemisch gemäß Herstellungsbeispiel 1 wurde in dem nachfolgend beschriebenen Fall höchst erfolgreich der Behandlung einer durch Sepsis indizierten Gerinnungsstörung angewandt.

Anamnese: Eine 59-jährige Patientin litt an starker durch Infektion mit Staphylococcus Epidermis und Candida Glabrata verursachter Sepsis, was unter anderem ein Hyperfibrinolysis-Syndrom bzw. eine disseminierte intravasale Gerinnung zur Folge hatte. Daraus resultierte eine weitverbreitete Verstopfung von Blutgefäßen, insbesondere der venösen und arteriellen Blutbahnen im rechten und linken Kopf- und Halsbereich (u.a. A. Carotis, Sinusvene, Sinus Cavernosus, V. Jugularis ext., V. Subclavia re.).

Die Behandlung erfolgte medikamentös mit Clindamycin und Metronidazol für Behandlungsabschnitte von jeweils zwei Wochen (davon eine überschneidend). Nachdem Resistenzen festgestellt wurden, erfolgte anschließend eine zweiwöchige Kombinationsbehandlung mit Vacomycin und Caspofungin. Gleichzeitig wurde der Patientin Heparin verabreicht.

Nach dem erfolglosen Ende dieser Behandlung verschlechterte sich der ACT-Wert der Patientin innerhalb eines Tages dramatisch, und stieg über einen Zeitraum von 17 Stunden von etwa 350 auf 700 an. Drei Tage später war dieser Gerinnungswert bereits im nicht messbaren Bereich von jenseits 1000.

Von diesem Ausgangswert konnte durch orale Gabe eines erfindungsgemäßen flüssigen Stoffgemisches bereits nach einer Stunde eine Reduktion des ACT-Wertes auf 600 erreicht werden, was geradezu als sensationelles Ergebnis betrachtet werden kann. Bereits nach sieben Minuten beginnend war eine Reduktion zu beobachten. Nach 2 Stunden war der Wert auf 457 gesunken.

Dieses Phänomen konnte vielfach reproduziert werden.

### Anwendungsbeispiel 2:

Es konnte gezeigt werden, dass das erfindungsgemäße Stoffgemisch gemäß Herstellungsbeispiel 1 sowohl bei Koagulopathien auf die Blutgerinnung einen regulativen, normalisierenden Einfluss hat.

Im Rahnem der Koagulopathie bei Sepsis wurde wiederholt eine disseminierte intravasale Gerinnung (DIC) dokumentiert mit ACT-Werten von 999 (nicht messbar), welches in den Kontrollen einem entsprechend erhöhten INR-Wert entsprach. Durch Gabe des erfindungsgemäßen Stoffgemischs konnte diese Situation innerhalb von 5-10 min normalisiert werden.

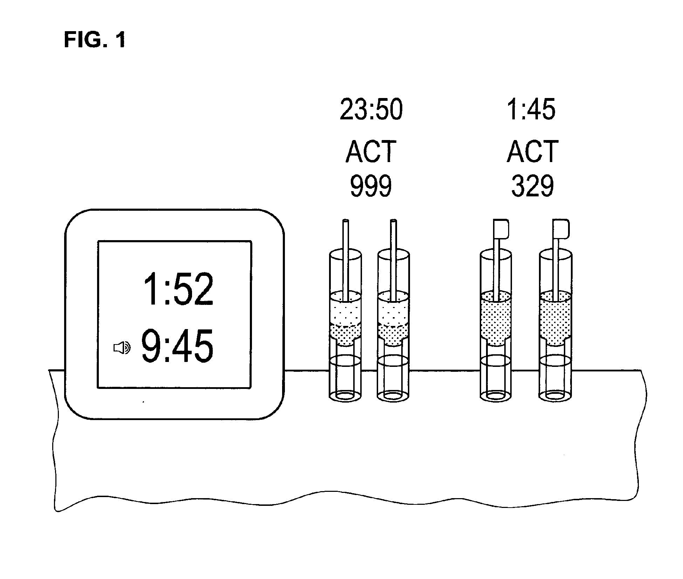

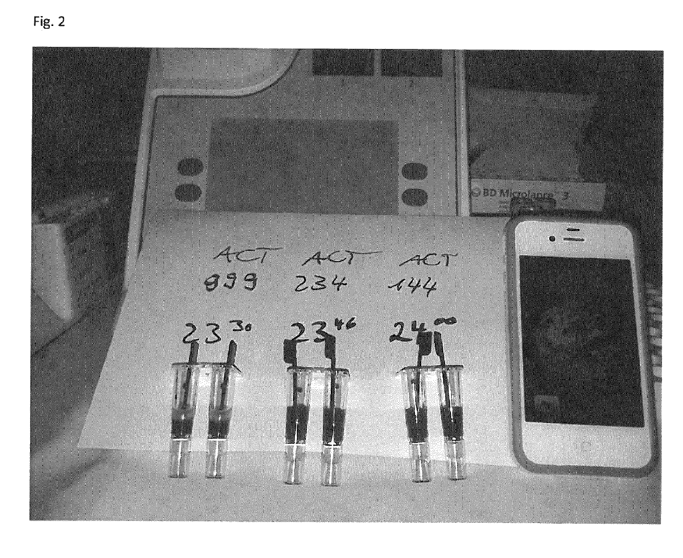

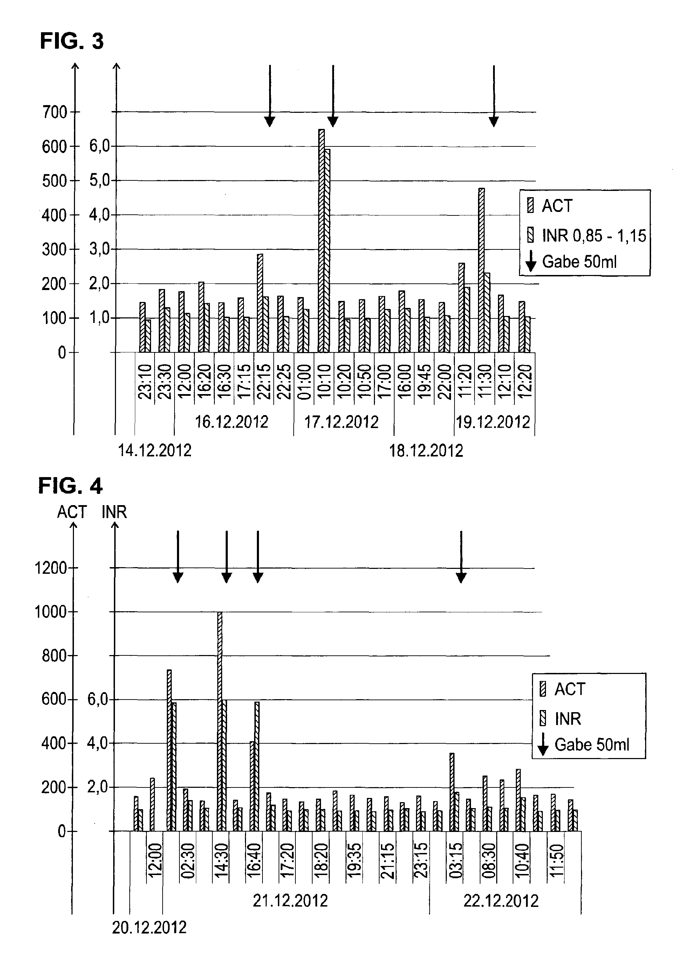

In Figur 1 ist eine Küvette gezeigt, welche unter der DIC das sofort in zwei Phasen zerfallende Blut zeigt und darauffolgend die normalisierten Gerinnungsergebnisse, die über die Zeit hin sich noch weiter normalisierten. Dieser Effekt konnte wiederholt reproduziert werden und ist mit dem INR korreliert, siehe in diesem Zusammenhang Figur 2 sowie die Tabellen gemäß Figuren 3 und 4.

Somit konnte gezeigt werden, dass bei Koagulopathien eine Normalisierung der Gerinnungssituation wiederholt erzielt wurde.

### Anwendungsbeispiel 3:

Es gibt erste Hinweise, dass auch im Rahmen der Hämophilie es zu positiven Effekten kommt. Dabei sollte das erfindungsgemäße Stoffgemisch nicht als Dauertherapie verabreicht werden, sondern z. B. einen Tag vor der regulären Blutgerinnungsfaktorengabe oder im Rahmen einer akuten oder bereits stattgehabten Blutung, die bisher mit dem Faktor nicht effektiv behandelt werden konnte.

### Anwendungsbeispiel 4:

Es gibt ferner Hinweise, dass bei Knochenbrüchen mit größeren Einblutungen, die über 3 Monate nach Trauma keine Heilungstendenzen zeigten, durch die Gabe des erfindungsgemäßen Stoffgemischs gemäß Herstellungsbeispiel 1 die ossäre Heilung induziert und vervollständigt wurde.

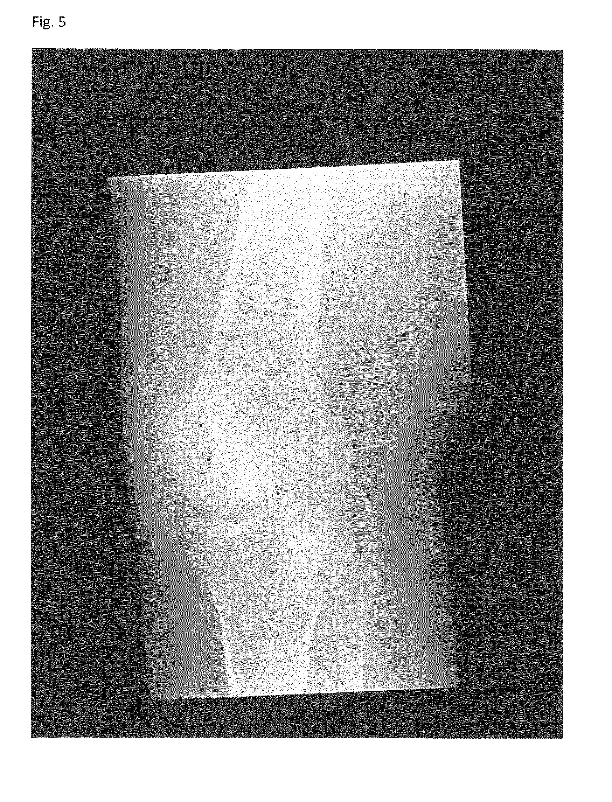

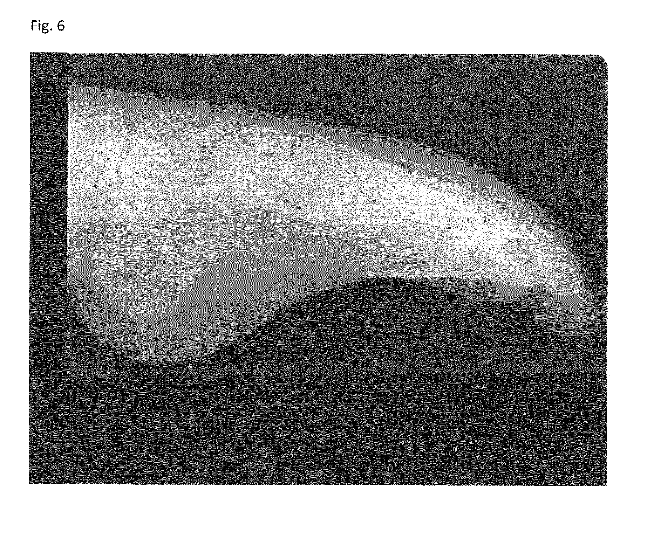

In Figur 5 ist ein Röntgenbild eines Kniegelenks einer 59 jährige Patientin mit lateraler Tibiakopf-Impressionsfraktur gezeigt. Figur 6 zeigt das Röntgenbild eines Fußes der selben Patientin, wobei eine Kalkaneus-Fraktur erkennbar ist.

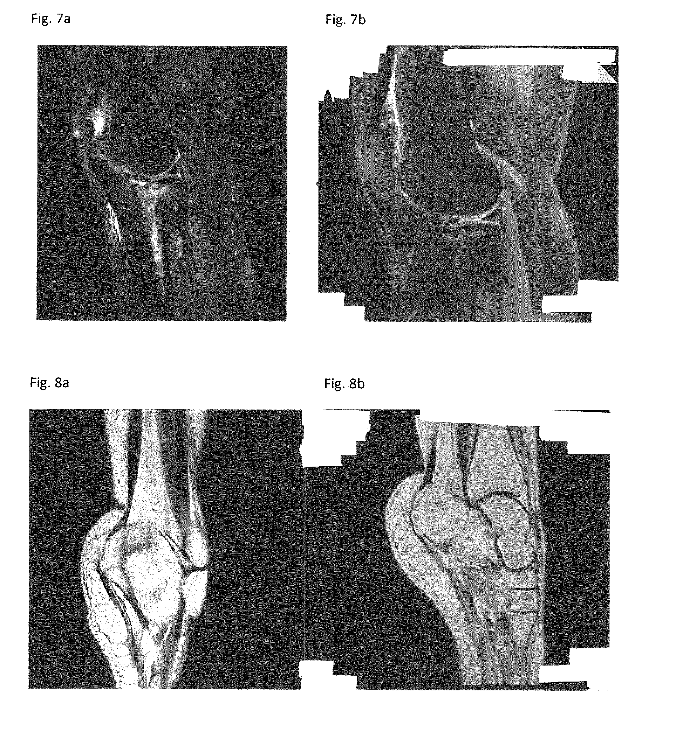

Aufgrund der Knochenfrakturen hatte die Patientin 2 ½ Monaten weiterhin persistent mit starken therapieresistenten Schmerzen. Es erfolgte daher eine MRT-Diagnostik, welche gemäß Figur 7 zeigte, dass sich in der lateralen Tibiakondyle (linkes Kniegelenk) eine waagerechte Fraktur ca. 1cm unter dem Gelenkniveau sowie eine senkrechte Fraktur medial der Metaphyse findet. Die Frakturen sind nicht disloziert, jedoch umgeben von Ödem im Knochenmark. Normale Menisken, Kreuzband und Ligamente. Es findet sich eine moderate Ödembildung und Faltenansammlung in der Bursa suprapatellaris. Aufgrund von Paraplegie liegt in Figur 6 eine Schrägaufnahme des linken Fußes vor. Es zeigt sich eine Fraktur des Kalkaneus mit Ödem im Knochenmark, disloziert um einige Millimeter nach medial. Ansonsten gutachterlich normales Fußgelenk.

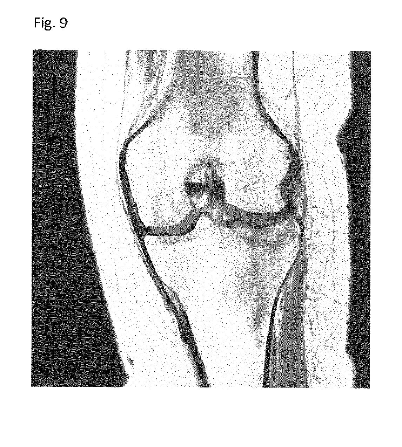

Wegen der ausbleibenden Heilungstendenzen nach 2 ½ Monaten dokumentiert in der MRT-Diagnostik gemäß Figuren 7 (Sagittale, axiale und coronare T1-, Protonen- und T2-gewichtete Bilder, TSE Technik, mit und ohne Fettsuppression, 3 Tesla Bildspeicherung in unserem PACS) und 8 (64-Zeilen-Dual-Source-CT, axial mit coronalen und sagittalen Rekonstruktionen, Bildarchivierung in unserem PACS) wurden weiterführende Maßnahmen inklusive Operation angedacht. Nach Therapie mit dem erfindungsgemäßen Stoffgemisch war weitere zwei Monate später der Befund nicht nur klinisch deutlich gebessert, ohne Schmerzen, ohne Schwellung, sondern auch im MRT diese erfreuliche Besserung nachweisbar. Dies ist mit Blick auf das Kniegelenk in Figur 9 erkennbar. Es war keine analgetische Medikation mehr notwendig und eine vollständige Abschwellung und eine weitgehende Wiederherstellung der Bewegungsfähigkeit konnte erreicht werden.

Der Befund des Kniegelenks nach zweimonatiger Behandlung unter Verwendung des erfindungsgemäßen Stoffgemischs war derart, dass im Bereich des lateralen Tiblaplateaus noch ein diskretes Restödem zu erkennen war, das deutlich rückläufig im Vergleich zu den Voraufnahmen war. Die Frakturlinien zeigten eine gute knöcherene Durchbauung. Geringer Impression am ventralen lateralen Tiblaplateau. Der darüber liegende Knorpel imprimierte ohne umschriebenen Defekt. Intakte Menisci und Bandstrukturen. Geringer Gelenkserguss. Die periartikulären Weichteile unauffällig. Dies kann so beurteilt werden, dass ein Zustand nach lateraler Tiblaplateaufraktur mit zunehmender knöcherner Durchbauung und rückläufigen Knochenmarksödem und geringer Impression am ventralen lateralen Tiblaplateau vorliegt. Ein geringer Gelenkserguss konnte beobachtet werden.

Der Befund des Kniegelenks nach zweimonatiger Behandlung unter Verwendung des erfindungsgemäßen Stoffgemischs war derart, dass ein Zustand nach Trümmerfraktur des Calcaneus mit guter knöcherner Durchbauung und sklerosierten Frakturlinien im dorsalen Calcaneusdrittel beobachtet werden konnte. Kein disloziertest Knochenfragment war nachzuweisen. Keine posttraumatische Arthrose im Subtalargelenk wurde erkannt. Das obere Sprunggelenk hatte keine osteochondrale Läsion. Die Tarsalia war kleinfleckig osteoporotisch aufgehellt.

Auch in anderen Fällen konnten intraartikuläre Blutungen oder prolongierte Verläufe nach Frakturen ohne adäquate Heilungstendenz mittels des erfindungsgemäßen Stoffgemischs zur Restitutio ad integrum geführt werden.

Es wird angenommen, dass auch bei Hämophilie ähnliche Ergebnisse sowohl auf der koagulatorischen als auch bei bereits vorhandenen oder akut auftretenden Komplikationen wie Einblutungen in Gewebe wie Knochen oder Gelenke u.a. mit Erfolg angewendet werden könnte.

Aus den bisherigen Untersuchungen und Ergebnissen wurde deutlich, dass das erfindungsgemäße Stoffgemisch sowohl auf die Physiologie der Gerinnung im Stande ist einzuwirken im Sinne einer Normalisierung der Gerinnung als auch bei der Förderung der Heilung nach Knochen und Gelenkstraumata/Einblutungen.

Allgemein ist hervorzuheben, dass die getestete erfindungsgemäße Aminosäure/Ribose Lösung in seiner Zusammensetzung in der Lage zu sein scheint, Veränderungen auf der Zellebene von ossären Strukturen herbei zu führen, wo reguläre Heilungsvorgänge außer Kraft gesetzt zu sein scheinen. Man muss dabei von einem sehr wirkungsvollen bzw. allgemein verwendbaren Effekt sprechen, der bei Verwertung in einem klinischen Setting positive Auswirkungen auf die desolate Situation von Patienten mit Antikörperbildung gegen die benötigten Gerinnungsfaktoren haben könnte.

Eine bevorzugte Verabreichung umfasst 10-30ml oral einerseits als Zusatzpräparat 24h vor der Gabe von dem benötigten Faktoren-Konzentrat, andererseits im akuten Zustand bei Komplikationen als auch zur Verbesserung der Wirkung der ossären/artikulären Heilungsvorgänge.

Somit könnte das erfindungsgemäße Stoffgemisch bei Hämophilie-Patienten, welche unter derartigen Komplikationen mit und ohne Trauma leiden, eingesetzt werden.

### Anwendungsbeispiel 5:

Auch für das erfindungsgemäße Stoffgemisch gemäß Herstellungsbeispiel 2 konnte ein regulativer, normalisierender Einfluss aus Koagulopathien bzw. auf die Blutgerinnung beobachtet werden.

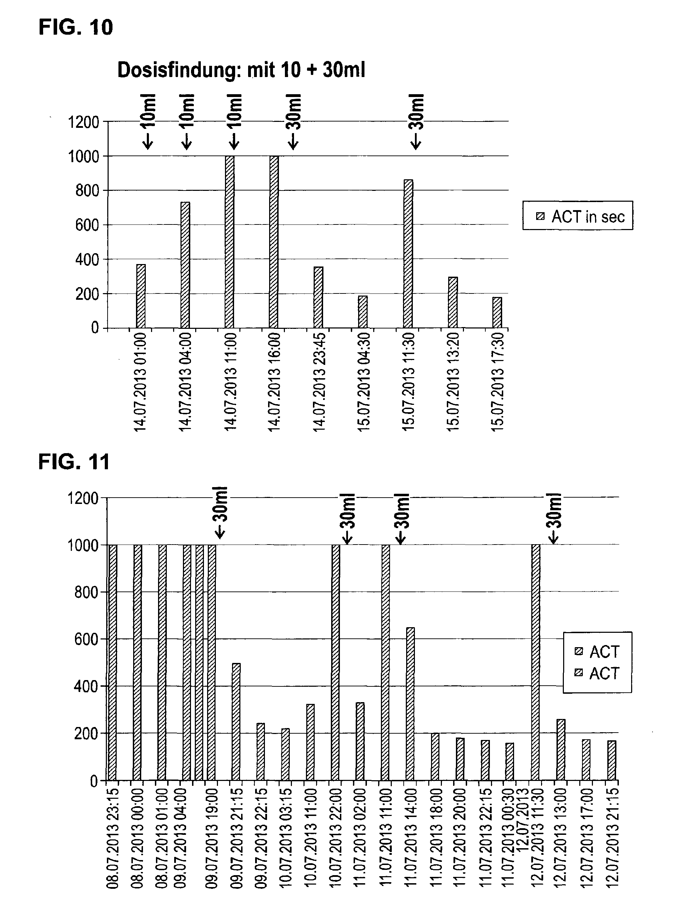

Ähnlich zu den Tabellen in Figuren 3 und 4 zeigen Figuren 10 und 11 einen reproduzierbaren Effekt, der mit dem INR korreliert ist.

### Wirkungsweise:

Zum Verständnis der Wirkungsweise des erfindungsgemäßen Stoffgemischs bei Koagulopathien wurden die bisherigen Ergebnisse der extrinsischen Gerinnung mit Verlängerung der ACT und des INR und der Normalisierung der beiden gezeigt. Ausführungen bezüglich der Initiierung der extrinsischen Gerinnung über Tissue-Factors und Faktor VII, welche direkt zu einer normalen Gerinnungssituation führen, unabhängig wie die dazwischenliegenden Faktoren arbeiten ist Gegenstand momentaner Untersuchungen. Dabei spielt das Verständnis der Toll-like Rezeptoren (TLR) eine große Rolle. Das hängt damit zusammen, dass Tissuefactor (der bei der Initiation der extrinsischen Gerinnungskaskade als gewebeständiger Faktor eine massgebliche Rolle spielt regulatorische Effekte zu erwarten sind in Zusammenhang mit den TLR.

Der Toll like Rezeptor ist gewebeständig und sitzt auf der Oberfläche von Leukozyten und Zellen der Lunge und Leber und des Intestinums. Er kann körperfremde Stoffe, insbesondere Bestandteile der Zellwand von Bakterien erkennen und entsprechende Signale an die Zelle weiterleiten. Mutationen im TLR-2-Gen des Menschen können Anfälligkeit für Lepra- oder Tuberkulose-Infektionen hervorrufen.

Anhand eines gnotobiotischen Mausmodell wurde daran geforscht, inwieweit Gerinnungsfaktoren durch Kolonisierung des Darms mit mikrobiellen Gemeinschaften verändert werden. Dabei spielt das Membranprotein Toll-like Rezeptor eine große Rolle, welcher ein Bestandteil des angeborenen Immunsystems und bereits bei den frühen Evolutionsstufen wie den Chordatieren zu finden ist. Er findet sich neben dem Epithel der Lunge und Leber auch auf der Mucosa der Darmschleimhaut. Es wurde gezeigt, dass die Darmflora u.a. über die Veränderung der Dichte der Toll-like Rezeptoren 1 und 2 ein Einfluss auf bestimmte Gerinnungsfunktionen ein Einfluss vorhanden ist. Dabei waren diese Effekte gerade über Dünndarmstrukturen darstellbar, welche sich alle vier Tage erneuern und die in Zusammenhang mit den Darmbakterien vermittelt werden. Die dort ansässige Darmflora ist massgebend für das vasculäre Remodeling und andererseits wird mit Hilfe von Toll-like Rezeptoren (TLR's) die microbial-associated molecular patterns (MAMP's) abgetastet und die Angiogenese gfördert. Dabei hat insbesondere Neuropilin-1 (Nrp-1) einen wesentlichen Einfluss auf die physiologische und pathologische Angiogenese und ist ein Regulator für VEGFR-2, welcher ein Co-Rezeptor für Plexin-dependent binding of class 3 Semaphorins (Sema) ist.

Bei konventionell aufgezogenen Mäusen mit physiologischer Darmflora (CONV-R) erhöhen sich TLR2 und sein Co-Rezeptor TLR1 von der Geburt an im Vergleich zu germ-free-Mäusen (GF). Dieser Effekt verminderte sich, wenn die Darmbakterien durch Antibiotikatherapie minimiert wurden. Eine gesteigerte TLR-1 und TLR-2 Expression konnte auch durch die Stimulation von TLR-2-Agonisten an der Dünndarm-Epithel-Zellinie MODE-K gezeigt werden. Bei konventionellen Mäusen (CONV-R) und ex-GF Mäusen, die für 14 Tage mit Darm-Mikrobiotika kolonisiert wurden (conventional-derived, CONV-D) verglichen mit GF-Kontrollen waren die Nrp-1-Spiegel reduziert. Zusätzlich wurde die Expression von Sema 3F und seinem Rezeptor Plexin A4 durch die Mikrobiotika reguliert und die epitheliale Zell-Proliferation durch MAMP's erhöht.

Es wurde also deutlich, dass TLR2/Nrp-1 Signaltransduktionswege durch die intestinalen Mikrobiotika reguliert werden und sie das intestinale Dünndarm-Remodeling beeinflussen.

Die aktuelle Forschung beschäftigt sich intensiv damit, wie die genauen Zusammenhänge zu verstehen sind, dass Darmbakterien direkt das Gerinnungssystem des Wirtes beeinflussen. Die Gerinnung wird dabei als eine Antwort des Wirtes auf die bakteriellen Herausforderungen gesehen. Dabei spielt der Membrangebundene Tissue-Faktor (TF) als Initiator für die Gerinnung eine massgebliche Rolle. Insbesondere in der intestinalen Mucosa ist TF an der Initiierung und der Gerinnungsfaktor Signal-Übertragung und Angiogenese beteiligt. Dadurch also, dass innerhalb der ersten 4 Lebenswochen des Neugeborenen vergleichbar mit der Kolonisation der Darmbakterien im Mausmodell sich die entscheidenden Veränderungen abspielen, wird nicht nur die erste Prägung des Immunsystems vorgenommen und auch die letztendliche Blutgruppeneigenschften vollständig festgelegt, sondern auch die Blutgerinnung eingestellt. Daher ist es bekannt, dass bei Neugeborenen innerhalb der ersten Lebenswochen eine u.a. erhöhte PTT physiologisch ist, welche sich im Verlauf reguliert.

Untersuchen im humanen System haben gezeigt, dass das erfindungsgemäße Stoffgemisch über die Regulation des Toll-like Rezeptor 1 bei oraler Applikation arbeitet und dieser wie bekannt gerade über das Dünndarmepithel reguliert wird.

Da der Toll-like Rezeptor aber auch Oberfläche von Leukozyten und Zellen der Lunge und Leber zu finden ist, wo ebenfalls entsprechende Signale über körperfremde Stoffe wie den MAMP's (Bestandteile der Zellwand von Bakterien) an die Zelle weitergeleiten werden, hat das erfindungsgemäße Stoffgemisch noch weitere Indikationsgebiete. Es gibt erste Hinweise, dass das erfindungsgemäße Stoffgemisch neben seiner Wirkung der Resistenzbrechung bei ESBL bildenden E.coli auch auf multiresistente Tuberkulose und auch Lepra-Infektionen vielversprechende Veränderungen bewirkt und Patienten wieder in kürzerer Zeit effektiv antibiotisch behandelt werden können.

Insbesondere im Zusammenhang mit langanhaltender Antibiotikatherapie von Intensivstations-patienten, wo die physiologische Darmflora massiv eingeschränkt war, konnte gezeigt werden, dass die Mykosen-Sekundärinfektionen massiv verringert wurden und auch erhöhte ACT und INR-Werte durch die Therapie anhand des erfindungsgemäßen Stoffgemischs deutlich gebessert wurden. Da es sich um eine Regulation des Toll-like Rezeptors handelt, konnten auch die Effekte einer Koagulopathie korrigiert werden, was die effektive Behandlung einer Disseminierten intravasalen Gerinnung ermöglichte.

Zusammenfassend kann aus den obengenannten Ergebnissen postuliert werden, dass insbesondere in den ersten vier Wochen nach der Geburt sich die entscheidenden Veränderungen im Aufbau der intestinalen Darmflora abspielen und dies in dem o.g. Einfluss auf die Dünndarmepithelien einen maßgeblichen Effekt auf die Normalisierung der Blutgerinnung hat, da bei den Neugeborenen innerhalb dieser Zeit die anfänglich physiologisch erhöhte PTT sich normalisiert.

Mit dem erfindungsgemäßen Stoffgemisch steht eine Substanz zur Verfügung, die einen regulatorischen Effekt in beiderlei Hinsicht aufweist, da es durch die Regulation von TLR-1 das Gleichgewicht der Interaktion von TLR-1 und TLR-2 herstellt und damit die vulnerable Phase überbrückt, bis der Organismus selber dieses Gleichgewicht nach der Umstellungsphase selber reguliert. Dabei hat das erfindungsgemäße Stoffgemisch durch seinen regulatorischen Effekt die Auswirkung, sowol eine verlängerte als auch eine verkürzte PTT in den Normalbereich zu führen, wie bereits an Patienten nach langer Antibiotikatherapie auf Intensivstation mit intravasaler disseminierter Gerinnung gezeigt werden konnte.

Durch orale Applikation von 5-10ml des erfindungsgemäßen Stoffgemischs könnte ein Rückgang der ICB bei neonatalen Kindern gezeigt werden im Vergleich zur Kontroll-Gruppe. Eventuell ist mit einem vorzeitigen Abbruch der Untersuchung zu rechnen, falls die Kontrollgruppe ein signifikant schlechteres Abschneiden aufweist und auch ihr diese Therapie durch den signifikanten Effekt nicht vorenthalten werden darf. Gerade bei Neugeborenen bietet sich der Einsatz des erfindungsgemäßen Stoffgemischs an, weil der Einsatz mit besonders niedrigem Risiko behaftet ist und bisher keinerlei Nebenwirkungen beobachtet werden konnten.

## Patentansprüche

1. Stoffgemisch enthaltend Ribose und die Aminosäuren Alanin und Glycin zur Verwendung als Arzneimittel oder Nahrungsergänzungsmittel zur Behandlung von Sepsis und/oder Koagulopathien, insbesondere von durch Sepsis induzierten Koagulopathien.

2. Stoffgemisch enthaltend Ribose und die Aminosäuren Alanin und Glycin zur Verwendung als Arzneimittel oder Nahrungsergänzungsmittel zur Behandlung von Knochenheilungsstörungen und/oder Knochenblutungen.

3. Stoffgemisch nach Anspruch 1 oder 2, **dadurch gekennzeichnet, dass** es sich bei der Ribose um D-Ribose handelt und/oder wobei es sich bei dem Alanin um DL-α-Alanin handelt und/oder wobei das relative Massenverhältnis von Ribose, Alanin und Glycin A:B:C mit 35 < A < 200, 20 < B < 80 und 1 < C < 40 beträgt, vorteilhafterweise mit 60<A<120, 30<B<70 und 2<C<20, weiterhin vorteilhafterweise mit A = 70, B = 40 und C = 4.

4. Stoffgemisch nach einem der vorangegangenen Ansprüche, **dadurch gekennzeichnet, dass** das Stoffgemisch weiterhin die Aminosäure Glutamin enthält, und wobei vorteilhafterweise das relative Massenverhältnis von Ribose, Alanin, Glycin und Glutamin A:B:C:D mit 35 < A < 200, 20 < B < 80, 1 < C < 40 und 20 < D < 80 beträgt, vorteilhafterweise mit 60<A<120, 30< B <70, 2 < C < 20 und 30 < D < 60, weiterhin vorteilhafterweise mit A = 70, B = 40, C = 4 und D = 50.

5. Stoffgemisch nach einem der vorangegangenen Ansprüche, **dadurch gekennzeichnet, dass** das Stoffgemisch weiterhin eine oder mehrere der Aminosäuren Serin, Valin, Leucin, Isoleucin, Cystein, 5-Hydroxy-Tryptophan und Tryptophan enthält, und wobei vorteilhafterweise das relative Massenverhältnis von Ribose, Alanin, Glycin und dem jeweiligen Stoff A:B:C:D beträgt mit 35 < A < 200, 20 < B < 80 und 1 < C < 40 beträgt, vorteilhafterweise mit 60<A<120, 30<B<70 und 2<C<20, weiterhin vorteilhafterweise mit A = 70, B = 40 und C = 4, und für:
Serin 10 < D < 150, vorteilhafterweise 40 < D < 120, weiterhin vorteilhafterweise D = 100;
Valin 40 < D < 150, vorteilhafterweise 60 < D < 120, weiterhin vorteilhafterweise D = 70;
Leucin 5 < D < 80, vorteilhafterweise 10 < D < 40, weiterhin vorteilhafterweise D = 30;
Isoleucin 1 < D < 80, vorteilhafterweise 1 < D < 60, weiterhin vorteilhafterweise D = 40;
Cystein 1 < D < 150, vorteilhafterweise 5 < D < 120, weiterhin vorteilhafterweise D = 10;
5-Hydroxy-Tryptophan 1 < D < 100, vorteilhafterweise 10 < D < 60, weiterhin vorteilhafterweise D = 50; und
Tryptophan 1 < D < 100, vorteilhafterweise 10 < D < 60, weiterhin vorteilhafterweise D = 50.

6. Stoffgemisch nach einem der vorangehenden Ansprüche, **dadurch gekennzeichnet, dass** das Stoffgemisch weiterhin eine oder mehrere der Aminosäuren Asparagin, Arginin, Prolin, Lysin enthält, wobei vorteilhafterweise das relative Massenverhältnis von Ribose, Alanin, Glycin und dem jeweiligen Stoff A:B:C:D beträgt mit 35 < A < 200, 20 < B < 80 und 1 < C < 40 beträgt, vorteilhafterweise mit 60<A<120, 30<B<70 und 2<C<20, weiterhin vorteilhafterweise mit A = 70, B = 40 und C = 4, und für:
Asparagin 1 < D < 100, vorteilhafterweise 10 < D < 80, weiterhin vorteilhafterweise 20 <= D <= 70;
Arginin 1 < D < 100, vorteilhafterweise 2 < D < 60, weiterhin vorteilhafterweise 4 <= D <= 40;
Prolin 1 < D < 100, vorteilhafterweise 5 < D < 80, weiterhin vorteilhafterweise 10 <= D <= 70;
Lysin 10 < D < 80, vorteilhafterweise 30 < D < 50, weiterhin vorteilhafterweise D = 40.

7. Stoffgemisch nach einem der vorhergehenden Ansprüche, **dadurch gekennzeichnet, dass** es sich um ein flüssiges Stoffgemisch handelt, das Ribose, Alanin und Glycin und ggf. die weiteren Stoffe in wässriger Lösung enthält, wobei die wässrige Lösung vorteilhafterweise
zwischen 35 g/l und 200 g/l, vorzugsweise zwischen 60 g/l und 120 g/l Ribose,
zwischen 20 g/l und 80 g/l, vorzugsweise zwischen 30 g/l und 70 g/l Alanin, und
zwischen 1 g/l und 40 g/l, vorzugsweise zwischen 2 g/l und 20 g/l Glycin enthält.

8. Stoffgemisch nach Anspruch 5, **dadurch gekennzeichnet, dass** es sich um ein flüssiges Stoffgemisch handelt, das Ribose, Alanin und Glycin und die eine oder mehreren weiteren Aminosäuren in wässriger Lösung enthält, wobei die wässrige Lösung vorteilhafterweise die jeweiligen Bestandteile in folgender Konzentration enthält:
Ribose zwischen 35 g/l und 200 g/l, vorzugsweise zwischen 60 g/l und 120 g/l;
Alanin zwischen 20 g/l und 80 g/l, vorzugsweise zwischen 30 g/l und 70 g/l;
Glycin zwischen 1 g/l und 40 g/l, vorzugsweise zwischen 2 g/l und 20 g/l;
Glutamin zwischen 20 g/l und 80 g/l, vorteilhafterweise zwischen 30 g/l und 60 g/l, weiterhin vorteilhafterweise 50 g/l;
Serin: zwischen 10 g/l und 150 g/l, vorteilhafterweise zwischen 40 g/l und 120 g/l, weiterhin vorteilhafterweise 100 g/l;
Valin zwischen 40 g/l und 150 g/l, vorteilhafterweise zwischen 60 g/l und 120 g/l, weiterhin vorteilhafterweise 70 g/l;
Leucin zwischen 5 g/l und 80 g/l, vorteilhafterweise zwischen 10 g/l und 40 g/l, weiterhin vorteilhafterweise 30 g/l;
Isoleucin zwischen 1 g/l und 80 g/l, vorteilhafterweise zwischen 1 g/l und 60 g/l, weiterhin vorteilhafterweise 40 g/l;
Cystein zwischen 1 g/l und 150 g/l, vorteilhafterweise zwischen 5 g/l und 120 g/l, weiterhin vorteilhafterweise 10 g/l;
5-Hydroxy-Tryptophan zwischen 1 g/l und 100 g/l, vorteilhafterweise zwischen 10 g/l und 60 g/l, weiterhin vorteilhafterweise 50 g/l;
Tryptophan zwischen 1 g/l und 100 g/l, vorteilhafterweise zwischen 10 g/l und 60 g/l, weiterhin vorteilhafterweise 50 g/l.

9. Stoffgemisch nach Anspruch 6, **dadurch gekennzeichnet, dass** es sich um ein flüssiges Stoffgemisch handelt, das Ribose, Alanin und Glycin und die eine oder mehreren weiteren Aminosäuren in wässriger Lösung enthält, wobei die wässrige Lösung vorteilhafterweise die jeweiligen Bestandteile in folgender Konzentration enthält:
Ribose zwischen 35 g/l und 200 g/l, vorzugsweise zwischen 60 g/l und 120 g/l;
Alanin zwischen 20 g/l und 80 g/l, vorzugsweise zwischen 30 g/l und 70 g/l;
Glycin zwischen 1 g/l und 40 g/l, vorzugsweise zwischen 2 g/l und 20 g/l;
Arginin zwischen 1 g/l und 100 g/l, vorteilhafterweise zwischen 2 g/l und 60 g/l, weiterhin vorteilhafterweise zwischen 4 g/l und 40 g/l;
Asparagin zwischen 1 g/l und 100 g/l, vorteilhafterweise zwischen 10 g/l und 80 g/l, weiterhin vorteilhafterweise zwischen 20 g/l und 70 g/l;
Prolin zwischen 1 g/l und 100 g/l, vorteilhafterweise zwischen 5 g/l und 80 g/l, weiterhin vorteilhafterweise zwischen 10 g/l und 70 g/l;
Lysin zwischen 10 g/l und 80 g/l, vorteilhafterweise zwischen 30 g/l und 50 g/l, weiterhin vorteilhafterweise 40 g/l.

10. Stoffgemisch enthaltend Ribose und die Aminosäuren Glycin, Prolin, Lysin, Arginin und Asparagin zur Verwendung als Arzneimittel oder Nahrungsergänzungsmittel zur Behandlung von Sepsis und/oder Koagulopathien, insbesondere von durch Sepsis induzierten Koagulopathien.

11. Stoffgemisch enthaltend Ribose und die Aminosäuren Glycin, Prolin, Lysin, Arginin und Asparagin zur Verwendung als Arzneimittel oder Nahrungsergänzungsmittel zur Behandlung von Knochenheilungsstörungen und/oder Knochenblutungen.

12. Stoffgemisch nach Anspruch 11, **dadurch gekennzeichnet, dass** das relative Massenverhältnis von Ribose, Glycin, Prolin, Lysin, Arginin und Asparagin A:B:C:D:E:F beträgt, mit den folgenden Werten:
35 < A < 200, vorteilhafterweise 60<A<120, weiterhin vorteilhafterweise A = 70;
1 < B < 40, vorteilhafterweise 5 < B < 20, weiterhin vorteilhafterweise B = 10;
10 < C < 100, vorteilhafterweise 20 < C < 80, weiterhin vorteilhafterweise 40 <= C <= 60;
20 < D < 80, vorteilhafterweise 30 < D < 60, weiterhin vorteilhafterweise D = 40;
1 < E < 100, vorteilhafterweise 2 < E < 60, weiterhin vorteilhafterweise 4 <= E <= 40;
10 < F < 80, vorteilhafterweise 20 < F < 50, weiterhin vorteilhafterweise F = 30.

13. Stoffgemisch nach einem der Ansprüche 11 oder 12, **dadurch gekennzeichnet, dass** es sich um ein flüssiges Stoffgemisch handelt, das Ribose, Glycin, Prolin, Lysin, Arginin und Asparagin in wässriger Lösung enthält, wobei die wässrige Lösung vorteilhafterweise die jeweiligen Bestandteile in folgender Konzentration enthält:
Ribose zwischen 35 g/l und 200 g/l, vorteilhafterweise zwischen 60<A<120, weiterhin vorteilhafterweise A = 70;
Glycin zwischen 1 g/l und 40 g/l, vorteilhafterweise zwischen 5 g/l und 20 g/l, weiterhin vorteilhafterweise B = 10 g/l;
Prolin zwischen 10 g/l und 100 g/l, vorteilhafterweise zwischen 20 g/l und 80 g/l, weiterhin vorteilhafterweise zwischen 40 g/l und 60 g/l;
Lysin zwischen 20 g/l und 80 g/l, vorteilhafterweise zwischen 30 g/l und 60 g/l, weiterhin vorteilhafterweise D = 40 g/l;
Arginin zwischen 1 g/l und 100 g/l, vorteilhafterweise zwischen 2 g/l und 60 g/l, weiterhin vorteilhafterweise zwischen 4 g/l und 40 g/l;
Asparagin zwischen 10 g/l und 80 g/l, vorteilhafterweise zwischen 20 g/l und 50 g/l, weiterhin vorteilhafterweise F = 30 g/l.

14. Stoffgemisch nach einem der vorhergehenden Ansprüche, **dadurch gekennzeichnet, dass** es sich bei den verwendeten Aminosäuren um rekristallisierte Aminosäuren handelt, vorzugsweise um wenigstens sechs mal rekristallisierte Aminosäuren, und/oder dass das Stoffgemisch ausschließlich die genannten Bestandteile aufweist.

15. Infusions- oder Trinklösung zur Verwendung als Arzneimittel oder Nahrungsergänzungsmittel Sepsis, Koagulopathien, Knochenheilungsstörungen und/oder Knochenblutungen, **dadurch gekennzeichnet, dass** die Infusions- oder Trinklösung die Inhaltsstoffe eines flüssigen Stoffgemisches nach Anspruch 6, 7, 8 oder 11 in 10-facher bis 100-facher, vorteilhafterweise in 25-facher bis 50-facher Verdünnung enthält.
